# EUROPEAN PATENT APPLICATION

(11) **EP 4 130 029 A1**
(43) Date of publication of application: **08.02.2023**
(21) Application number: 21190005.5
(22) Date of filing: 05.08.2021
(51) Int. Cl.: C07K 14/74, A61K 38/17, A61P 35/00, C07K 16/28

(54) **PHARMACEUTICAL COMPOSITIONS COMPRISING HLA FUSION PROTEINS**

(71) Applicant: ImmunOs Therapeutics AG, 8952 Schlieren (CH)
(72) Inventor: MARROQUIN BELAUNZARAN, Osiris, 8952 Schlieren (CH); RAFIEI, Anahita, 8952 Schlieren (CH); KUMAR, Anil, 8952 Schlieren (CH); RENNER, Christoph, 8952 Schlieren (CH)
(74) Representative: Schulz Junghans Patentanwälte PartGmbB

(57) **Abstract**

The invention relates to pharmaceutical compositions comprising HLA fusion proteins for use in treating neoplastic disease. The invention also provides combination medicaments comprising both HLA fusion proteins and checkpoint inhibitors, for use in treating cancer.

## Description

The present invention relates to pharmaceutical compositions for use as antineoplastic medicaments, said compositions comprising an HLA fusion protein associated with a beta-2-microglobulin polypeptide, and having desirable immunomodulatory and recombinant protein expression properties.

### Background of the Invention

Classical MHC-I molecules (also known as HLA-I in humans) are dimeric or trimeric structures comprising a membrane-bound heavy chain characterized by an extracellular domain (comprising an α1, α2, and an α3 domain), usually bound non-covalently to a β2-microglobulin (β2m) light chain, and optionally, a small peptide epitope associated with the peptide-binding cleft. MHC Class I molecules may also disassociate into free heavy chains lacking β2m, and/or peptide epitope, referred to as open conformers (Arosa et al. Open conformers: the hidden face of MHC-I molecules, Trends in Immunology 2007 Mar; 28(3):115-23). The inventors have identified selected HLA, particularly the HLA-B57 haplotype linked to certain immune correlates in HIV infection, as promising immunomodulatory medicaments when delivered as a fusion protein with a stabilizing peptide such as an immunoglobulin (Ig) Fc portion, where the HLA is an open conformer lacking association with β2m, or a peptide epitope in the HLA binding cleft (see WO 2017153438 A1).

To obtain isolated open conformer HLA used in previous iterations of the invention, the HLA heavy chain fusion protein / β2m complex is separated, for example under acidic conditions, prior to purification, for example by size-based chromatography, prior to refolding of the isolated HLA heavy chain fusion proteins into open conformers. However, while HLA heavy chain open conformers, such as the HLA-B57 heavy chain-derived fusion protein studied herein, have desirable immunomodulatory qualities, upscaling of manufacturing to industrial quantities has revealed challenges. Up to half of the HLA fusion protein is lost during the process of separating the immunomodulatory HLA fusion protein from the β2m.

Based on the above-mentioned state of the art, the objective of the present invention is to provide an improved pharmaceutical composition of an HLA heavy chain-based fusion protein with favorable immunomodulatory properties for use treating malignant neoplastic diseases. This objective is attained by the subject-matter of the independent claims of the present specification, with further advantageous embodiments described in the dependent claims, examples, figures and general description of this specification.

### Summary of the Invention

By investigating the immunological qualities of an intermediate, HLA heavy chain fusion protein / β2m polypeptide complex in comparison to an open conformer HLA heavy chain lacking β2m polypeptide, the inventors made the surprising observation that the HLA fusion protein / β2m polypeptide complex molecule also exhibits desirable immunomodulatory properties, and delivers an increased beneficial therapeutic effect in treating cancer, particularly in a humanized immune system model of cancer.

A first aspect of the invention relates to a pharmaceutical composition for use in treating malignant neoplastic disease, said composition comprising an HLA fusion protein (comprising an HLA heavy chain extracellular domain polypeptide and a stabilizing Ig Fc polypeptide), where the HLA fusion protein is additionally associated with a β2m polypeptide, and where the compositions contain at least one pharmaceutically acceptable carrier, diluent or excipient. In particular embodiments, the HLA heavy chain portion of the HLA fusion protein is the extracellular domain of a naturally-occurring HLA heavy chain selected from HLA-B57, HLA-C08, HLA-A25, HLA-B58, HLA-B27, HLA-A30, HLA-B53, or HLA-C12. In other particular embodiments, the HLA fusion protein comprises a variant HLA heavy chain polypeptide, particularly a variant of the HLA-B57 protein, where the variant has at least 95% similar to one of the HLA heavy chains listed above, and retains a similar biological function.

Further embodiments of the pharmaceutical composition for use according to the invention relate to non-covalent association of the HLA fusion protein with the β2m polypeptide, variant HLA-B57 heavy chain polypeptides, and Ig Fc polypeptides, peptide linkers, and secretion signals of particular utility for inclusion in such pharmaceutical compositions, as well as combination medicaments further comprising a checkpoint inhibition agent.

Another aspect of the invention is a checkpoint inhibitor agent for use in treating a malignant neoplastic disease in a patient in combination with a pharmaceutical composition comprising an HLA fusion protein according to the first aspect of the invention.

### Detailed Description of the Invention

### Terms and definitions

For purposes of interpreting this specification, the following definitions will apply and whenever appropriate, terms used in the singular will also include the plural and vice versa. In the event that any definition set forth below conflicts with any document incorporated herein by reference, the definition set forth shall control.

The terms "comprising," "having," "containing," and "including," and other similar forms, and grammatical equivalents thereof, as used herein, are intended to be equivalent in meaning and to be open ended in that an item or items following any one of these words is not meant to be an exhaustive listing of such item or items, or meant to be limited to only the listed item or items. For example, an article "comprising" components A, B, and C can consist of (i.e., contain only) components A, B, and C, or can contain not only components A, B, and C but also one or more other components. As such, it is intended and understood that "comprises" and similar forms thereof, and grammatical equivalents thereof, include disclosure of embodiments of "consisting essentially of" or "consisting of."

Where a range of values is provided, it is understood that each intervening value, to the tenth of the unit of the lower limit, unless the context clearly dictate otherwise, between the upper and lower limit of that range and any other stated or intervening value in that stated range, is encompassed within the disclosure, subject to any specifically excluded limit in the stated range. Where the stated range includes one or both of the limits, ranges excluding either or both of those included limits are also included in the disclosure.

Reference to "about" a value or parameter herein includes (and describes) variations that are directed to that value or parameter per se. For example, description referring to "about X" includes description of "X."

As used herein, including in the appended claims, the singular forms "a," "or," and "the" include plural referents unless the context clearly dictates otherwise.

Unless defined otherwise, all technical and scientific terms used herein have the same meaning as commonly understood by one of ordinary skill in the art (e.g., in cell culture, molecular genetics, nucleic acid chemistry, hybridization techniques and biochemistry). Standard techniques are used for molecular, genetic and biochemical methods (see generally, Sambrook et al., Molecular Cloning: A Laboratory Manual, 4th ed. (2012) Cold Spring Harbor Laboratory Press, Cold Spring Harbor, N.Y. and Ausubel et al., Short Protocols in Molecular Biology (2002) 5th Ed, John Wiley & Sons, Inc.) and chemical methods.

The term *polypeptide* in the context of the present specification relates to a molecule consisting of 50 or more amino acids that form a linear chain wherein the amino acids are connected by peptide bonds. The amino acid sequence of a polypeptide may represent the amino acid sequence of a whole (as found physiologically) protein or fragments thereof. The term "polypeptides" and "protein" are used interchangeably herein and include proteins and fragments thereof. Polypeptides are disclosed herein as amino acid residue sequences.

Amino acid residue sequences are given from amino to carboxyl terminus. Capital letters for sequence positions refer to L-amino acids in the one-letter code (Stryer, Biochemistry, 3rd ed. p. 21). Lower case letters for amino acid sequence positions refer to the corresponding D- or (2R)-amino acids. Sequences are written left to right in the direction from the amino to the carboxy terminus. In accordance with standard nomenclature, amino acid residue sequences are denominated by either a three letter or a single letter code as indicated as follows: Alanine (Ala, A), Arginine (Arg, R), Asparagine (Asn, N), Aspartic Acid (Asp, D), Cysteine (Cys, C), Glutamine (Gln, Q), Glutamic Acid (Glu, E), Glycine (Gly, G), Histidine (His, H), Isoleucine (Ile, I), Leucine (Leu, L), Lysine (Lys, K), Methionine (Met, M), Phenylalanine (Phe, F), Proline (Pro, P), Serine (Ser, S), Threonine (Thr, T), Tryptophan (Trp, W), Tyrosine (Tyr, Y), and Valine (Val, V).

The terms *gene expression* or *expression,* or alternatively the term *gene product,* may refer to either of, or both of, the processes - and products thereof - of generation of nucleic acids (RNA) or the generation of a peptide or polypeptide, also referred to transcription and translation, respectively, or any of the intermediate processes that regulate the processing of genetic information to yield polypeptide products. The term *gene expression* may also be applied to the transcription and processing of an RNA gene product, for example a regulatory RNA or a structural (e.g. ribosomal) RNA. If an expressed polynucleotide is derived from genomic DNA, expression may include splicing of the mRNA in a eukaryotic cell. Expression may be assayed both on the level of transcription and translation, in other words mRNA and/or protein product.

In the context of the present specification, the terms *sequence identity, sequence similarity* and *percentage of sequence identity* refer to a single quantitative parameter representing the result of a sequence comparison determined by comparing two aligned polypeptide sequences position by position. Methods for alignment of sequences for comparison are well-known in the art. Alignment of sequences for comparison may be conducted by the local homology algorithm of Smith and Waterman, Adv. Appl. Math. 2:482 (1981), by the global alignment algorithm of Needleman and Wunsch, J. Mol. Biol. 48:443 (1970), by the search for similarity method of Pearson and Lipman, Proc. Nat. Acad. Sci. 85:2444 (1988) or by computerized implementations of these algorithms, including, but not limited to: CLUSTAL, GAP, BESTFIT, BLAST, FASTA and TFASTA. Software for performing BLAST analyses is publicly available, e.g., through the National Center for Biotechnology-Information (http://blast.ncbi.nlm.nih.gov/).

One example for comparison of amino acid sequences is the BLASTP algorithm that uses the default settings: Expect threshold: 10; Word size: 3; Max matches in a query range: 0; Matrix: BLOSUM62; Gap Costs: Existence 11, Extension 1; Compositional adjustments: Conditional compositional score matrix adjustment. Unless stated otherwise, sequence identity values provided herein refer to the value obtained using the BLAST suite of programs (Altschul et al., J. Mol. Biol. 215:403-410 (1990)) using the above identified default parameters for protein.

Reference to identical sequences without specification of a percentage value implies 100% identical sequences (i.e. the same sequence).

As used herein, the term *pharmaceutical composition* refers to an HLA fusion protein associated with β2m according to the invention, together with at least one pharmaceutically acceptable carrier. In certain embodiments, the pharmaceutical composition according to the invention is provided in a form suitable for topical, parenteral or injectable administration.

As used herein, the term *pharmaceutically acceptable carrier* includes any solvents, dispersion media, coatings, surfactants, antioxidants, preservatives (for example, antibacterial agents, antifungal agents), isotonic agents, absorption delaying agents, salts, preservatives, drugs, drug stabilizers, binders, excipients, disintegration agents, lubricants, sweetening agents, flavoring agents, dyes, and the like and combinations thereof, as would be known to those skilled in the art (see, for example, Remington: the Science and Practice of Pharmacy, ISBN 0857110624).

As used herein, the term *treating* or *treatment* of any disease or disorder (e.g. cancer) refers in one embodiment, to ameliorating the disease or disorder (e.g. slowing or arresting or reducing the development of the disease, or at least one of the clinical symptoms thereof, for example, slowing, or reducing tumor growth). In another embodiment "treating" or "treatment" refers to alleviating or ameliorating at least one physical parameter including those which may not be discernible by the patient. In yet another embodiment, "treating" or "treatment" refers to modulating the disease or disorder, either physically, (e.g., stabilization of a discernible symptom), physiologically, (e.g., stabilization of a physical parameter), or both. Methods for assessing treatment and/or prevention of disease are generally known in the art, unless specifically described hereinbelow.

In the context of the present specification, the term *peptide linker,* or *amino acid linker* refers to a polypeptide of variable length that is used to connect two polypeptides in order to generate a single chain polypeptide. Exemplary embodiments of linkers useful for practicing the invention specified herein are oligopeptide chains consisting of 1, 2, 3, 4, 5, 10, 20, 30, 40 or 50 amino acids. A non-limiting example of an amino acid linker is the polypeptide GGGGSGGGGS (SEQ ID NO 003) that links an HLA heavy chain polypeptide with a stabilizing peptide, for example, linking an HLA-B57 with a IgG4 Fc polypeptide in an HLA fusion protein.

The term *Human leukocyte antigen (HLA) heavy chain, HLA heavy chain* in the context of the present specification relates to the protein encoded by an *MHC Class I histocompatibility antigen* gene, particularly a classical, MHC class 1a heavy chain. In humans, an HLA heavy chain can be a monomer, or form a part of dimeric structures comprising a heavy chain with three extracellular domains (α1, α2, and α3), bound non-covalently to a β2m light chain, or optionally, trimeric structures wherein a small peptide is associated at the peptide-binding cleft. Full length *HLA heavy chain* polypeptides comprise an extracellular domain comprising an α1, an α2, and an α3 domain, a transmembrane domain, and an intracellular domain.

A list of naturally occurring *HLA heavy chains* which are considered embodiments of the term according to this aspect of the invention are listed in Table 1.

**Table 1: List of HLA heavy chain alleles**

| **HLA-A** | **HLA-B** | | **HLA-C** |
|---|---|---|---|
| A01 | B07 | B53 | C01 |
| A02 | B08 | B54 | C02 |
| A03 | B13 | B55 | C03 |
| A11 | B14 | B56 | C04 |
| A23 | B15 | B57 | C05 |
| A24 | B18 | B58 | C06 |
| A25 | B27 | B59 | C07 |
| A26 | B35 | B67 | C08 |
| A29 | B37 | B73 | C12 |
| A30 | B38 | B78 | C14 |
| A31 | B39 | B81 | C15 |
| A32 | B40 | B82 | C16 |
| A33 | B42 | B83 | C17 |
| A34 | B44 | | C18 |
| A36 | B46 | | |
| A43 | B47 | | |
| A66 | B48 | | |
| A68 | B49 | | |
| A69 | B50 | | |
| A74 | B51 | | |
| A80 | B52 | | |

The term *variant* in the context of the present specification relates an HLA heavy chain polypeptide sequence with at least one amino acid residue that differ from a naturally-occurring polypeptide sequence. For example, a variant HLA heavy chain polypeptide in which one, or several amino acid substitutions have been introduced, such that it differs from the original, naturally occurring human protein sequence it is derived from.

The term *extracellular domain* as applied to an HLA heavy chain, or a variant of an HLA heavy chain in the context of this specification, refers to the extracellular portion of an HLA heavy chain polypeptide which extends from the cell surface. The extracellular portion of an HLA Class 1a polypeptide comprises the alpha (α) 1 domain, α2 domain, and α3 domain, which comprise regions essential for receptor ligand interactions which mediate the immunomodulatory effects of the HLA fusion protein in the pharmaceutical composition for use according to the invention. The *extracellular domain* excludes the transmembrane domain, and the intracellular domain, though it may optionally further comprise:
- a secretory signal, for example, the initial 24 amino acids present in most naturally occurring HLA polypeptides annotated as a signal peptide, or an alternative secretion signal designed for efficient HLA fusion protein secretion, such as SEQ ID NO 004, and/or
- C-terminal to the alpha 1, alpha 2 and alpha 3 domains of the extracellular domain of an HLA heavy chain, the initial 6 residues of the "connecting peptide" region joining the alpha 3 domain, with the transmembrane domain, and present in most naturally occurring full-length HLA heavy chains, such as, for example, HLA-B57.

In the context of the present specification, the term *HLA fusion protein* refers to a recombinant polypeptide which comprises the extracellular domain of an HLA heavy chain, joined to a stabilizing domain, particularly a stabilizing immunoglobulin (Ig) Fc, optionally by means of a peptide linker. The term encompasses such an *HLA fusion protein* in complex with a β2-microglobulin polypeptide as secreted from mammalian cell culture, as well as purified open conformer *HLA fusion protein* which is not associated with β2-microglobulin. The term *HLA fusion protein* may refer to a monomer comprising a single HLA polypeptide joined to a single stabilizing immunoglobulin (Ig) Fc domain, or a dimer formed by association of a first HLA fusion protein monomer, and a second HLA fusion protein monomer, particularly joined via their Ig Fc domains.

In the context of the present specification, the term *β2-microglobulin* (*β2m, B2m), B2m polypeptide,* or *β2m polypeptide* refers to the beta (β) chain, also known as the HLA light chain of MHC class I molecules. The term *β2-microglobulin* encompasses firstly, a pre-processing *β2-microglobulin* comprising a secretory signal, for example, the sequence of Uniprot P61769, or the sequence SEQ ID NO 006, and secondly, the post-secretion form of the protein, in which a secretory signal portion of the protein has been removed by cleavage during the secretion process, as found in an HLA fusion protein : β2m polypeptide complex comprised within the pharmaceutical composition according to the invention.

In the context of the present specification, the terms *open conformer (OC), open conformer HLA fusion protein, open conformer HLA,* or *open conformer HLA heavy chain* refer to an HLA heavy chain molecule, particularly in the context of an HLA fusion protein further comprising a stabilizing polypeptide, lacking association with a β2m molecule. In the context of the present specification, the terms *HLA-B57.OC,* or *HLA-B57.OC*^{*(A46E*/*V97R)*} refer to an *HLA-B57 open conformer* in the form of a fusion protein comprising an HLA-B57 heavy chain polypeptide linked to an IgG4 Fc polypeptide, lacking association with a β2m molecule.

In the context of the present specification, the term *secretory signal, secretory signal peptide* or *signal sequence* refers to an N-terminal leader sequence initiating the open reading frame (ORF) of a polypeptide, usually about 6-30 amino acids in length. In rare cases, a *secretory signal* is placed at the C-terminus of a polypeptide. *Secretory signals* are sometime referred to as targeting signals, localization signals, transit peptides, leader sequences, or leader peptides. *Secretory signals* which enable efficient secretion of a polypeptide from cells are well known, and may be included in the ORF of a recombinant protein in order to facilitate export of a polypeptide to the supernatant in cell-based polypeptide manufacturing system, allowing purification of a polypeptide from the cell supernatant. Upon translation of the mRNA encoding the *secretory signal,* it is recognized by a cytosolic protein mediating transfer of the mRNA-ribosome complex to a channel protein in the endoplasmic reticulum (ER). The newly synthesized polypeptide comprising the *secretory signal peptide* is translocated to the ER lumen through the channel protein, entering the cell secretion pathway. *Signal sequences* of particular use according to the invention are those that are cleaved from the final polypeptide product following translation, for example, SEQ ID NO 004.

In the context of the present specification, the terms *immunoglobulin crystallizable fragment (Fc) region,* or *Ig Fc* refers to a fraction of an antibody, or immunoglobulin (Ig), comprised of a C_{H}2 and a C_{H}3 domain. *Ig Fc* encompass both a monomer, or a dimer comprising two *Ig Fc,* covalently linked by disulfide bonds. In the context of the HLA fusion protein according to the invention, disulfide bonds can join two HLA fusion proteins molecules, each comprising *Ig Fc* domains. The presence of the *Ig Fc* in the HLA fusion protein facilitates increased solubility, stability, avidity, half-life, and from a technological point of view, cost-effective production and purification in mammalian systems (protein A or G purification).

In the context of the present *specification,* the term *checkpoint inhibitory agent* encompasses a cancer immunotherapy agent capable of disrupting an inhibitory signalling cascade that limits immune cell activation, and in particular T cell activation, known in the art as an immune checkpoint mechanism, particularly a *checkpoint inhibitor antibody* or an antibody-like molecule, such as an antibody fragment, a diabody, or a single variable chain antibody fragment. Examples of *checkpoint inhibitory agent* include, for example, an antibody, antibody like molecule, or natural ligand receptor which binds specifically to CTLA-4 (Uniprot P16410), PD-1 (Uniprot Q15116), PD-L1 (Uniprot Q9NZQ7), B7H3 (CD276; Uniprot Q5ZPR3), VISTA (Uniprot Q9H7M9), TIGIT (UniprotQ495A1), or TIM-3 (HAVCR2, Uniprot Q8TDQ0), CD137 (41BB, Uniprot Q07011), CD40 (Uniprot Q09LL4), CD27 (Uniprot P26841), OX40 (CD134, UniprotP43489), NKG2A (Uniprot P26715), CD86 (Uniprot P42081), CD80 (Uniprot P33681), LAG-3 (Uniprot P18627).

### Pharmaceutical composition comprising HLA fusion protein for treating cancer

A first aspect of the invention relates to a pharmaceutical composition for use treating a malignant, neoplastic disease, said composition comprising an HLA fusion protein, associated with a B2m polypeptide. Said HLA fusion protein according to the invention comprises
- a first polypeptide comprising the extracellular domain of an HLA heavy chain (particularly, including the extracellular α1, α2 and α3 domains), and
- a second stabilizing polypeptide, namely the Ig Fc portion of an immunoglobulin.

Using the β2m-associated HLA fusion protein confers the advantage of increased yields of the immunomodulatory HLA fusion protein, as producing this product does not incur the loss of protein that occurs during a β2m disassociation step (Fig. 2), and enhances certain immunological properties such as phagocyte uptake of tumor cells, in comparison to an open conformer HLA fusion protein alternative (Fig 4). Importantly, the pharmaceutical composition according to the invention comprising an HLA fusion protein and B2m is associated with enhanced survival when using a pharmaceutical composition according to the invention to treat cancer in humanized mice comprising human immune cells, compared to an open conformer format lacking β2m polypeptide (Fig 5).

In some embodiments of the pharmaceutical composition for use according to the invention, the β2m polypeptide is linked by a peptide linker to the HLA fusion protein.

In particular embodiments of the pharmaceutical composition for use according to the invention, the β2m polypeptide is non-covalently associated with said HLA fusion protein.

In particular embodiments of the pharmaceutical composition for use according to the invention, the HLA fusion protein is associated with a β2m molecule at a ratio of between 3:5 to 7:5, more particularly between 4:5 to 6:5. In other words, the HLA fusion protein and the β2m polypeptide are present at a ratio of, or close to, 1 to 1.

In particular embodiments of the pharmaceutical composition for use according to the invention, two HLA fusion proteins, each associated with a β2m polypeptide may be associated in a dimerized form via their Fc portions.

### HLA heavy chain polypeptides

Certain domains of HLA heavy chain proteins not required for cognate ligand interactions, are not included in the HLA polypeptide portion of the HLA fusion protein comprised in the pharmaceutical composition for use according to the invention. The intracellular domain, and the transmembrane domain are absent from the HLA heavy chain polypeptide. Structural data suggests that HLA heavy chains interact with ligands such as Killer immunoglobulin-like receptors (KIR) and leukocyte immunoglobulin-like receptors (LILR) via regions distant from the transmembrane region. Amino acids close to the membrane do not generally interact with receptors. Furthermore, the inventors surmise that the high content of hydrophobic amino acids within the 5 C-terminal amino acid motif of the extracellular domain connecting peptide of naturally occurring HLA heavy chain sequences, is likely to introduce undesirable properties into recombinant proteins, such as a tendency towards protein aggregation, which can then affect the production, purification, stability and toxicity in downstream production processes.

In particular of the pharmaceutical composition for use according to the invention, the HLA heavy chain extracellular domain polypeptide of the HLA fusion protein component comprises the core structure of the extracellular portion of an HLA heavy chain protein sequence, comprising the alpha 1, 2, and 3 domains, as this portion confers the HLA fusion protein with the ability to interact with surface molecules on target cells.

In some embodiments of the pharmaceutical composition for use according to the invention, the HLA heavy chain polypeptide portion of the HLA fusion protein has the polypeptide sequence of the extracellular domain of a naturally occurring HLA heavy chain listed in Table. 1. In particular embodiments, the HLA heavy chain extracellular domain is that of a naturally occurring HLA heavy chain with immunomodulatory qualities, such as specific binding to regulatory KIR3DL1, LILRA and LILRB1/2 cell surface proteins which alter innate and adaptive immune cell function. In some such embodiments, the HLA heavy chain portion of the HLA fusion protein is derived from, or essentially is, the extracellular domain of HLA-B58. In some such embodiments, the HLA heavy chain portion of the HLA fusion protein is derived from, or essentially is, the extracellular domain of HLA-B27. In other embodiments, the HLA heavy chain portion of the HLA fusion protein is derived from, or essentially is, the extracellular domain of HLA-B44. In further embodiments, the HLA heavy chain portion of the HLA fusion protein is derived from, or essentially is, the extracellular domain of HLA-B81. In other embodiments, the HLA heavy chain portion of the HLA fusion protein is derived from, or essentially is, the extracellular domain of HLA-C12. In particular embodiments, the HLA heavy chain portion of the HLA fusion protein is derived from, or essentially is, the extracellular domain of HLA-B57.

In particular embodiments of the pharmaceutical composition for use according to the invention, the HLA fusion protein comprises a variant HLA heavy chain extracellular domain polypeptide. Said variant HLA heavy chain polypeptide is characterized by a sequence similarity (on the protein level) of at least (≥) 95% to the non-variant extracellular domain of an HLA heavy chain, and having a similar biological activity compared to the original sequence in the context of an HLA fusion protein. In particular embodiments, the variant HLA heavy chain is ≥98% similar to the naturally occurring HLA heavy chain extracellular domain from which it is derived, while having a similar biological activity compared to the original sequence in the context of an HLA fusion protein.

A similar biological activity can be defined as at least 65% particularly 85%, or even 95% of the capacity of a variant HLA heavy chain polypeptide HLA fusion protein to bind to LILRB2, compared to the equivalent non-variant structure, as measured by an enzyme-linked immunosorbent assay (ELISA) method. This may be assessed by calculating the EC50, i.e. the concentration of a fusion protein that gives a half-maximal response, in this case, half the maximal binding to a biotinylated LILRB2 molecule. For example, for representative HLA-B57 non-variant structure in the example, theEC50 or LILRB2 binding is approximately 21nM. The threshold for EC50 for a suitable variant as measured by ELISA with approximately 65% similar biological function is therefore approximately 32nM, 85% is approximately 29nM, and 95% is approximately 22nM. The variant HLA-B57 sequence has an EC50 of approximately 8, and is therefore above the threshold for biologically similar function (over 100%), as the EC50 is below, and therefore improved compared to the original sequence, however a variant HLA-B57 with an EC50 above 35nM would be excluded.

In other embodiments of the pharmaceutical composition according to the invention, the biological function of the HLA fusion protein open conformer bearing a variant HLA heavy chain polypeptide is enhanced relative to the equivalent non-variant control structure. In terms of ELISA measurement of the EC50 of maximal binding of the fusion protein to LILRB2, the HLA fusion protein open conformer comprising the variant HLA-B57^{(A46E/V97R)} polypeptide according to the invention, is lower than that of an HLA fusion protein bearing the wildtype of HLA-B57 molecule, such that a similar biological activity may be defined as at least 100%, particularly 200%, or even 250% of the HLA fusion protein open conformer comprising the non-variant polypeptide equivalent. In other words, the EC50 of the variant HLA fusion protein in terms of LILRB2 binding is less than approximately 21nM (100%), 10.5nM (200%), or even 8.3 nM (250%).

To determine the EC50 according to the invention, streptavidin coated high binding capacity 96 well plates may be coated with 50 µl of c-terminally biotinylated LILRB2 (for example, obtained from BPS Bioscience #100335) at a final concentration of 5 µg/ml in PBS buffer. PBS and IgG isotype may be used as negative controls. A serial dilution of HLA fusion protein is applied in a titrated series, (for example, eight concentration points: 10, 2.5, 1, 0.25, 0.1, 0.025, 0.01, 0.0025 µg/ml), preferably applied in 50ul duplicates. A labelled antibody that can detect the fusion protein (for example, an APC conjugated goat anti-human IgG antibody (Jackson Immuno Research #109-135-098, 1:100 dilution in TBS 50 µl) may then be applied to detect HLA fusion protein binding and background, and fluorescence excitation and emission measured at the appropriate wavelength (for example 650 nm & 660 nm). A three-parameter based log (agonist) model is one suitable means to determine the EC50 of the HLA fusion protein binding to the LILRB2 ELISA.

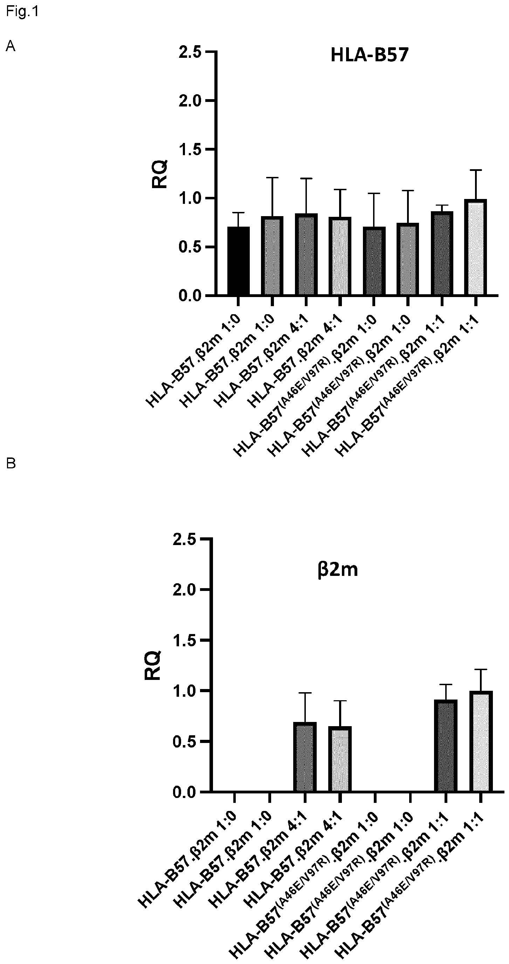

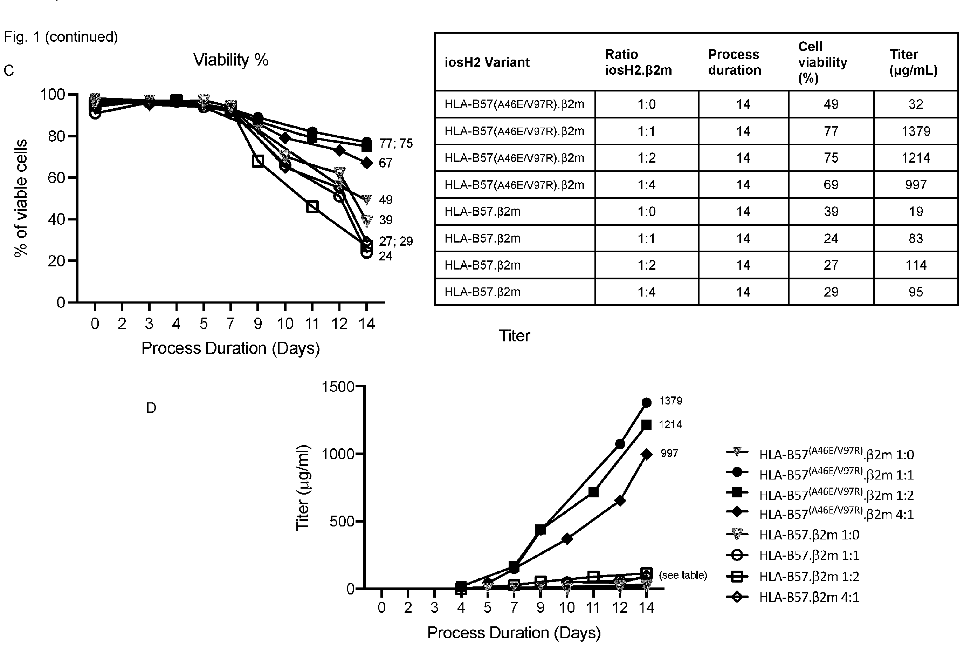

In particular embodiments of the pharmaceutical composition for use according to the invention, HLA fusion protein comprises a variant HLA-B57 heavy chain extracellular domain polypeptide, which is characterized by at least one, or two specific amino acid substitutions which differ from the polypeptide sequence a naturally-occurring HLA heavy chain extracellular domain polypeptide. The HLA-B57 heavy chain gene family currently encompasses 221 known variants with unique nucleic acid sequences, numbering HLA-B*57:01 to HLA-B*57:141, encoding several dozen unique protein sequences. The protein sequence for which are known, and may be retrieved, for example, by entering the search term "B*57" into the MGT/HLA Allele Query Form provided by the European Bioinformatics Institute Immuno Polymorphism Database, Robinson J. et al. 2013 Nucleic Acids Res. 41:D1234, https://www.ebi.ac.uk/ipd/imgt/hla/allele.html). In more particular embodiments, the HLA heavy chain polypeptide is a variant of the naturally occurring extracellular domain of an HLA-B57 heavy-chain polypeptide sequence, in which one, or two amino acid substitutions have been performed such that the variant HLA heavy chain polypeptide is characterized by an E at position 46, and an R at position 97. In other words, an amino acid other than E has been replaced with an E at position 46, and/or an amino acid other than R, has been replaced with an R at position 97. The numbering of these amino acids refers to the assignment of integers sequentially beginning with the G, S, H motif that initiates the extracellular domain of a secreted HLA-B57 portion, lacking the secretion signal, with the numbers 1, 2, and 3. The inventors find these amino acid substitutions correlate with high yields of the resulting variant-based HLA fusion protein compared to the wildtype sequence (Fig. 1), and a favorable LILRB2 binding profile.

In more particular embodiments of the pharmaceutical composition for use according to the invention, the HLA heavy chain extracellular polypeptide portion of the HLA fusion protein according to the invention comprises the variant HLA-B57 sequence designated SEQ ID NO 001. In still more particular embodiments, the HLA heavy chain polypeptide essentially consists of the sequence designated SEQ ID NO 001.

In certain embodiments of the pharmaceutical composition for use according to the invention, the HLA heavy chain polypeptide and the Ig Fc polypeptide are joined by a peptide linker. In particular embodiments, the peptide linker is between 5 and 20 amino acids in length. In more particular embodiments this joining peptide linker has the sequence SEQ ID NO 003.

In particular embodiments of the pharmaceutical composition for use according to the invention, the HLA fusion protein comprises a polypeptide with the sequence designated SEQ ID NO 005, and is associated with a β2m polypeptide. In other embodiments, the HLA fusion protein additionally comprises a secretory signal upstream of the sequence SEQ ID NO 005. In particular embodiments, the secretory signal upstream of the HLA fusion protein polypeptide is SEQ ID NO 004. In other embodiments, the HLA fusion protein essentially consists of a secretory signal of SEQ ID NO 004, joined to a polypeptide designated SEQ ID NO 005. In more particular embodiments of the pharmaceutical composition for use according to the invention, the HLA fusion protein essentially consists of the sequence designated SEQ ID NO 005 (comprising a variant extracellular domain of HLA-B57 fused to an IgG4 Fc), and is associated with a β2m polypeptide.

In particular embodiments of the pharmaceutical composition for use according to the invention, the β2m polypeptide associated with the HLA fusion protein has the sequence designated SEQ ID NO 006.

In alternative embodiments of the pharmaceutical composition for use according to the invention, the HLA fusion protein comprises an HLA-B57 heavy chain polypeptide identical to SEQ ID NO 001, other than a single differing amino acid residue (wherein the single differing amino acid residue is not position 46, or 97). In other words, in addition to the one, or in some cases, two amino acid substitutions at positions 46, and/or 97, the recombinant HLA-B57 polypeptide according to this embodiment includes at least one further amino acid substitution compared to the naturally occurring HLA-B57 molecule from which it is derived.

### Stabilizing and linking peptides

The HLA fusion portion of the pharmaceutical composition for use according to the invention, comprises a stabilizing polypeptide conferring stability during expression and purification. The presence of stabilizing portion of the HLA fusion protein increases the yield and solubility by reducing degradation and oligomerization of the HLA fusion protein, and further associated with improved viability of cells expressing the fusion protein.

In particular embodiments of the pharmaceutical composition for use according to the invention, the stabilizing polypeptide of the HLA fusion protein is a human Ig Fc polypeptide. An Ig Fc portion may also prolong the in vivo half-life of a molecule *in vivo* by binding to recycling receptors. In particular embodiments of the pharmaceutical composition for use according to the invention, the stabilizing polypeptide is an isotype IgG Fc. An IgG Fc stabilizing peptide domain delivers an added advantage during purification of the HLA fusion protein, by enabling absorption to a protein A or G coated surface. The inventors consider a possible alternative that may provide similar benefits if attached to the functional HLA polypeptides in lieu of Ig Fc could be bovine serum albumin. Previous work by the inventors has established that an albumin molecule, such as bovine serum albumin, may also serve as a stabilizing polypeptide. It is also known that PEGylation can enhance the half-life of proteins in circulation, and may feasibly serve as a stabilizing polypeptide according to the invention.

In particular embodiments of the pharmaceutical composition for use according to the invention, the HLA fusion protein comprises an IgG4 polypeptide, which is desirable isotype in therapeutic fusion proteins due to its low cytotoxicity. In still more particular embodiments of the pharmaceutical composition for use according to the invention, the HLA fusion protein comprises an altered IgG4 S228P.dk molecule with sequence SEQ ID NO 002. This is characterized by a mutation in the hinge region of the IgG4, where Proline (P) is substituted for serine (S) at position 228 of the original IgG4 antibody, and dK indicates a deletion of the last amino acid Lysine (K) on the original IgG4 sequence. These changes give the IgG4 format stability and less heterogenicity. Both changes are well established and used commonly in diverse Fc constructs.

In certain embodiments of the pharmaceutical composition for use according to the invention, the HLA fusion protein comprises an HLA polypeptide joined with an IgG polypeptide as part of a single polypeptide chain by a peptide linker, a short sequence of amino acids 5, 10, 15, or 20 residues in length.

In particular embodiments, the peptide linker is a non-immunogenic sequence, such as a sequence rich in serine and glycine residues. In more particular embodiments, the peptide linker has the sequence SEQ ID NO 003.

In particular embodiments of the pharmaceutical composition for use in treating cancer, it is in the format of a dimer. Said dimer comprises a first monomer and a second monomer, and each monomer independently of the other monomer comprises an HLA fusion protein, an HLA heavy chain extracellular domain polypeptide fused to an Ig Fc portion, the latter of which may associate via disulfide bonds. Each monomer according to the invention is additionally associated with a B2m polypeptide.

### Dosing and Administration of HLA fusion protein pharmaceutical compositions

The pharmaceutical composition for use in treating malignant neoplastic disease according to the present invention comprises an HLA fusion protein associated with B2m polypeptide, and is typically formulated into pharmaceutical dosage forms to provide an easily controllable dosage of the drug and to give the patient an elegant and easily handled product. Said pharmaceutical composition further contains a pharmaceutically acceptable carrier. In further embodiments, the composition comprises at least two pharmaceutically acceptable carriers, such as those described herein.

The dosage regimen for the pharmaceutical composition of the present invention will vary depending upon known factors, such as the pharmacodynamic characteristics of the particular agent and its mode and route of administration; the species, age, sex, health, medical condition, and weight of the recipient; the nature and extent of the symptoms; the kind of concurrent treatment; the frequency of treatment; the route of administration, the renal and hepatic function of the patient, and the effect desired. In certain embodiments, the pharmaceutical composition of the invention may be administered in a single daily dose, or the total daily dosage may be administered in divided doses of two, three, or four times daily.

Many procedures and methods for preparing pharmaceutical compositions are known in the art, see for example L. Lachman et al. The Theory and Practice of Industrial Pharmacy, 4th Ed, 2013 (ISBN 8123922892).

### Combination medicaments

Another aspect of the invention is pharmaceutical composition (comprising an HLA fusion protein : B2m polypeptide complex according to the first aspect of invention), formulated for administration in combination with a checkpoint inhibitory agent.

Another aspect of the invention is a checkpoint inhibitor agent for use in treating a malignant neoplastic disease, formulated for administration in combination with pharmaceutical composition comprising and HLA fusion protein according to the first aspect of the invention.

In particular embodiments of the pharmaceutical composition, or the checkpoint inhibitory agent according to the invention, the checkpoint inhibitory agent is capable of disrupting the inhibitory signaling cascade that limits immune cell activation, and in particular T cell activation. In certain embodiments, the checkpoint inhibitory agent is an antibody fragment, or an antibody-like molecule capable of binding to one of CTLA-4, PD-1, PD-L1, or PD-L2 with a dissociation constant of at least 10⁻⁷ M⁻¹.

Particularly, the checkpoint inhibitor agent is a non-agonist CTLA-4 ligand, a non-agonist PD-1 ligand, a non-agonist PD-L1 ligand, or a non-agonist PD-L2 ligand, which does not lead to attenuated T cell activity when binding to CTLA-4, PD-1, PD-L1 or PD-L2, respectively, on the surface on a T-cell. In certain embodiments, the term "non-agonist CTLA-4 ligand" or "non-agonist PD-1 ligand" covers both antagonists of CTLA-4 or PD-1 and ligands that are neutral vis-à-vis CTLA-4 or PD-1 signaling. In some embodiments, non-agonist CTLA-4 ligands used in the present invention are able, when bound to CTLA-4, to sterically block interaction of CTLA-4 with its binding partners CD80 and/or CD86 and non-agonist PD-1 ligands used in the present invention are able, when bound to PD-1, to sterically block interaction of PD-1 with its binding partners PD-L1 and/or PD-L2.

In particular embodiments, the checkpoint inhibitory agent disrupts inhibitory signaling cascades via a capacity to bind to CTLA-4 with a dissociation constant of at least 10⁻⁷ M⁻¹, 10^{- 8} M⁻¹ or 10⁻⁹ M⁻¹ and which inhibits the biological activity of its respective target. A non-agonist PD-1 ligand or a non-agonist PD-L1 (PD-L2) ligand in the sense of the invention refers to a molecule that is capable of binding to PD-1 (PD-L1, PD-L2) with a dissociation constant of at least 10⁻⁷ M⁻¹, 10⁻⁸ M⁻¹ or 10⁻⁹ M⁻¹ and which inhibits the biological activity of its respective target.

In further particular embodiments, the checkpoint inhibitory agent is a checkpoint inhibitor antibody selected from the clinically available antibody drugs ipilimumab (Bristol-Myers Squibb; CAS No. 477202-00-9), tremelimumab (CAS 745013-59-6), nivolumab (Bristol-Myers Squibb; CAS No 946414-94-4), pidilizumab (CAS No. 1036730-42-3), atezolizumab (Roche AG; CAS No. 1380723-44-3), avelumab (Merck KGaA; CAS No. 1537032-82-8), durvalumab (Astra Zeneca, CAS No. 1428935-60-7), and cemiplimab (Sanofi Aventis; CAS No. 1801342-60-8). In still more particular embodiments, the check point inhibitor agent is pembrolizumab (Merck Inc.; CAS No. 1374853-91-4).

The antibody fragment relating to a checkpoint inhibitor format may be a Fab domain or a variable fragment (Fv) domain of an antibody, or a single-chain antibody fragment, which is a fusion protein consisting of the variable regions of light and heavy chains of an antibody connected by a peptide linker. The checkpoint inhibitor may also be a single domain antibody, consisting of an isolated variable domain from a heavy or light chain. Additionally, an antibody may also be a heavy-chain antibody consisting of only heavy chains such as antibodies found in camelids. An antibody-like molecule may be a repeat protein, such as a designed ankyrin repeat protein (Molecular Partners, Zurich).

In certain embodiments, the combination therapy comprises two distinct dosage forms, for example, wherein said pharmaceutical composition comprising an HLA fusion protein is provided as a dosage form for intra-tumoral delivery, or local delivery in the vicinity of the tumor, for example, by subcutaneous injection, or intra-tumoral injection into a solid tumor, and said checkpoint inhibitor agent is provided as a dosage form for systemic delivery, particularly by intravenous injection. However, said checkpoint inhibitor agent and said pharmaceutical composition comprising an HLA fusion protein may also be delivered in two similar dosage forms.

Administration in combination, encompasses both simultaneous administration of the checkpoint inhibitor agent and the pharmaceutical composition comprising an HLA fusion protein, or in separate formulations, or administration of one substance immediately prior to, for example, in the week prior to, or at least in the month prior to, or immediately subsequent to, for example, in the week, or at most the month subsequent to, administration of a second substance.

### Medical treatment, Dosage Forms, Method of Manufacture

The pharmaceutical composition for use according to the invention is provided for use in treating malignant neoplastic disease, encompassing various forms of cancer. Pre-clinical studies on other forms of LILBR2-directed antineoplastic therapy (one of several mechanism through which the pharmaceutical composition according to the invention is predicted to act) have demonstrated efficacy in renal cancer, and ovarian cancer. The safety and tolerability of LILRB2-targeting antibody MK-4830 is currently under investigation in a clinical trial targeting a wide range of solid organ cancers (mesothelioma, triple negative breast cancer, ovarian cancer, lung cancer, glioblastoma, pancreatic cancer, gastric cancer), in combination with chemotherapy (ClinicalTrials.gov Identifier: NCT03564691), all of which the inventors consider may feasibly be treated effectively by a pharmaceutical composition according to the invention.

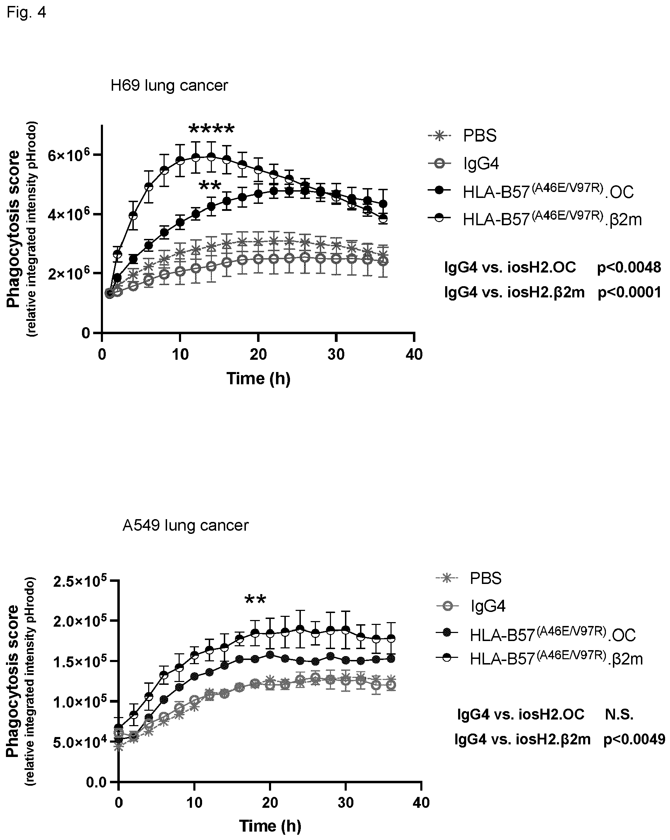

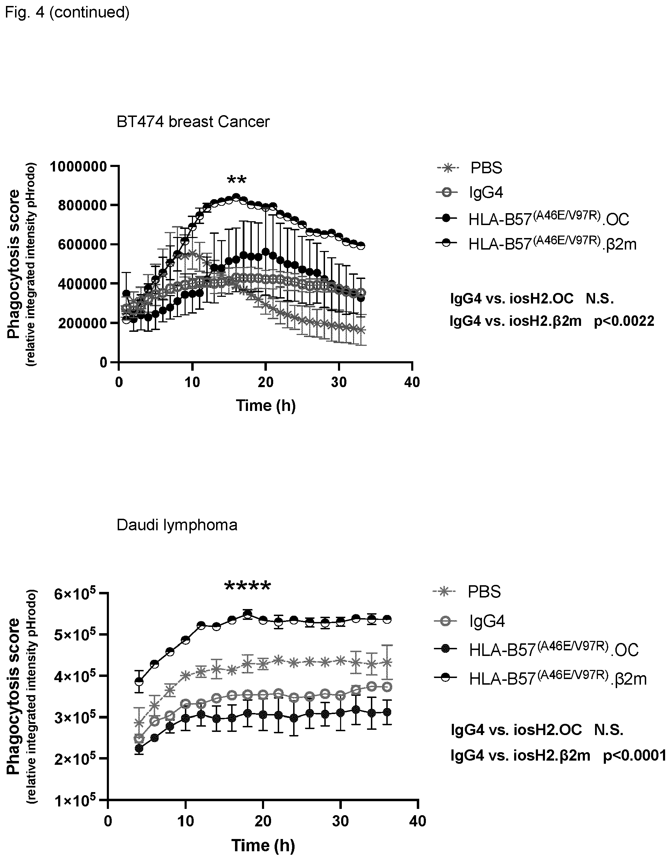

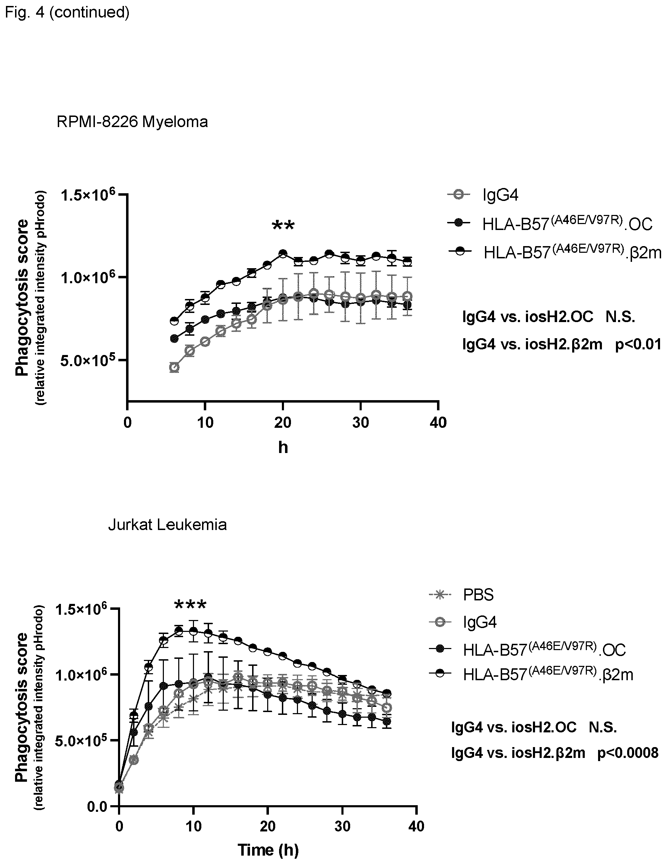

In particular embodiments of the pharmaceutical composition comprising an HLA fusion protein, it is provided for use to treat a type of liquid, or blood cancer. The inventors consider the characteristic T cell-exhaustion, and accessibility of circulating blood cancer cells to the T cell, and macrophage activation induced by pharmaceutical compositions according to the invention mean the treatment is likely to be effective. In particular such embodiments, the pharmaceutical composition is provided for use in a patient diagnosed with T cell leukemia. In other particular embodiments, the pharmaceutical composition is provided for use in a patient diagnosed with lymphoma, including, but not limited to, Burkitt's lymphoma as modelled by Daudi cells in the examples. In further embodiments, the pharmaceutical composition is provided for use in a patient diagnosed with multiple myeloma, as modelled by the RPMI-8226 cell line (Fig. 4).

In other particular embodiments, the pharmaceutical composition comprising an HLA fusion protein is provided for use in a patient diagnosed with a solid cancer, or a metastasis of a solid cancer. In some particular embodiments, the pharmaceutical composition is for use in a patient diagnosed with lung cancer. In particular embodiments, the lung cancer is a form of non-small cell lung cancer. In other particular embodiments, the lung cancer is a form of small cell lung cancer, or carcinoma. In other particular embodiments, the pharmaceutical composition comprising an HLA fusion protein is for use in a patient diagnosed with a form of breast cancer. In more particular embodiments, the cancer is estrogen receptor positive. In other particular embodiments, the cancer is progesterone receptor positive. In other particular embodiments, the cancer is human epidermal growth factor receptor 2 positive.

In some particular embodiments, the pharmaceutical composition comprising an HLA fusion protein, or the immune checkpoint inhibitor, is for use in a patient diagnosed with colon cancer. In particular embodiments, metastatic colon cancer.

In some embodiments of aspects of the invention relating to administration of a pharmaceutical composition according to the invention in combination with a checkpoint inhibition agent, it is provided for use in a form of malignant disease, or cancer, in which checkpoint inhibition therapy is approved for monotherapy, or combination therapy. In particular embodiments, the combination treatment is for use in a patient with colon cancer, particularly metastatic colon cancer. In other particular embodiments, the cancer is melanoma. In further particular embodiments, the cancer is pancreatic cancer. In still further particular embodiments the cancer is breast cancer.

Similarly within the scope of the present invention is a method of treating a cancer patient, comprising administering to the patient a pharmaceutical composition comprising an HLA fusion protein associated with a B2m polypeptide according to the invention, optionally in combination with a checkpoint inhibitory agent. The invention further encompasses, as an additional alternative aspect, the use of a pharmaceutical composition comprising an HLA fusion protein and beta-2-microglobulin, as specified in detail above, for use in a method of manufacture of a medicament for the treatment or prevention of cancer.

Dosage forms may be for parenteral administration such as subcutaneous, intravenous, intrahepatic or intramuscular injection forms. Optionally, a pharmaceutically acceptable carrier and/or excipient may be present.

Wherever alternatives for single separable features are laid out herein as "embodiments", it is to be understood that such alternatives may be combined freely to form discrete embodiments of the invention disclosed herein.

The invention is further illustrated by the following items:
A. An HLA fusion protein for use in the treatment of a malignant neoplastic disease, said HLA fusion protein comprising:
   i. a naturally-occurring MHC class 1a heavy chain extracellular domain polypeptide, or a variant M HC class 1 a heavy chain extracellular domain polypeptide,
      wherein the variant MHC class 1a heavy chain extracellular domain polypeptide differs by no more than one, or two amino acid substitutions from the polypeptide sequence of a naturally occurring MHC class 1a heavy chain extracellular domain polypeptide; and
   ii. an Ig Fc polypeptide, particularly an IgG Fc polypeptide, more particularly an IgG4 Fc polypeptide; and
   wherein the HLA fusion protein is associated with a beta 2 microglobulin (B2m) polypeptide.
B. The HLA fusion protein for use according to item A, wherein the naturally-occurring MHC class 1a heavy chain extracellular domain polypeptide is the extracellular domain of an MHC class Ia heavy chain polypeptide from an HLA heavy chain selected from B57, C08, A25, B58, B27, A30, B53, or C12.
C. The HLA fusion protein for use according to item A or B, wherein the variant MHC class 1a heavy chain extracellular domain polypeptide is a variant of a naturally occurring extracellular domain of an HLA-B57 polypeptide,
   and wherein the variant MHC class 1a heavy chain extracellular domain polypeptide is characterized by an E at position 46, and an R at position 97.
D. The HLA fusion protein for use according to any one of the items A to C, wherein the recombinant MHC class 1a heavy chain polypeptide comprises, or essentially consists of, the sequence SEQ ID NO 001.
E. The HLA fusion protein for use according to any one of the items A to D, comprising:
   a. a naturally-occurring MHC class 1a heavy chain extracellular domain polypeptide, or a variant MHC class 1a heavy chain extracellular domain polypeptide as specified in any one of the items A to D,
   b. an IgG Fc polypeptide, particularly an IgG4 F polypeptide c, more particularly an IgG4 Fc polypeptide with the sequence SEQ ID NO 002,
   c. a peptide linker connecting the recombinant HLA-B57 polypeptide to the Ig Fc polypeptide, particularly a peptide linker between 5 and 20 amino acids in length, more particularly a peptide linker with the sequence SEQ ID NO 003,
   and wherein optionally, the HLA fusion protein further comprises:
   d. a secretory signal, particularly wherein the secretory signal is 16 to 30 amino acids in length, more particularly wherein the secretory signal is removed by cleavage during the process of secretion from the cell, still even more particularly a secretory signal with the sequence SEQ ID NO 004.
F. The HLA fusion protein for use according to any one of the items A to E, wherein the HLA fusion protein comprises, or essentially consists of, the sequence designated SEQ ID NO 005.
G. The HLA fusion protein for use according to any one of the items A to F, wherein the HLA fusion protein is non-covalently associated with the B2m polypeptide at a ratio of between 3:5 to 7:5, more particularly between 4:5 to 6:5.
H. The HLA fusion protein for use according to any one of the items A to G, wherein the HLA is in the form of a dimer, comprising a first HLA monomer and a second HLA monomer, wherein
   said first HLA fusion protein monomer essentially consists of a first HLA fusion protein as specified in any one of the items A to G associated with a first B2m polypeptide; and
   said second HLA fusion protein monomer essentially consists of a second HLA fusion protein as specified in any one of the items A to G associated with a second B2m polypeptide, and particularly wherein the first and second HLA monomer are identical.
I. A pharmaceutical composition comprising an HLA fusion protein according to any one of the items A to H,
   particularly wherein the HLA fusion protein is non-covalently associated with a B2m polypeptide at a ratio of between 3:5 to 7:5, more particularly between 4:5 to 6:5.
J. The pharmaceutical composition comprising the HLA fusion protein according to item I, for use in the treatment of a malignant neoplastic disease.
K. A pharmaceutical composition for use according to item J,
   - wherein the pharmaceutical composition is administered in combination with a checkpoint inhibitory agent, particularly a checkpoint inhibitory agent selected from an antibody, an antibody fragment, or an antibody-like molecule,
   - particularly wherein said checkpoint inhibitory antibody is capable of binding to one of CTLA-4, PD-1, PD-L1, or PD-L2 with a dissociation constant of at least 10⁻⁷ M⁻¹,
   - and wherein said checkpoint inhibitory antibody is provided in a dosage form for systemic delivery.
L. A checkpoint inhibitory agent for use in the treatment of a malignant neoplastic disease,
   - wherein the checkpoint inhibitor is administered in combination with the HLA fusion protein for use according to any one of the items A to H, or the pharmaceutical composition according to any one of the items I to K,
   - wherein said checkpoint inhibitory antibody is capable of binding to one of CTLA-4, PD-1, PD-L1, or PD-L2 with a dissociation constant of at least 10⁻⁷ M⁻¹,
   - wherein said checkpoint inhibitory antibody is provided in a dosage form for systemic delivery,
   - and particularly wherein the checkpoint inhibitory agent is selected from an antibody, an antibody fragment, or an antibody-like molecule.

The invention is further illustrated by the following examples and figures, from which further embodiments and advantages can be drawn. These examples are meant to illustrate the invention but not to limit its scope.

### Description of the Figures

- Fig. 1: shows superior expression properties of HLA-B57^{(A46E/V97R)} IgG4 fusion protein. RNA profiles of the indicated (A) HLA-B57 IgG4 fusion proteins and (B) β2m expressed from vectors within CHO cell clones. Fusion protein expression from clones transfected with HLA B57.β2m (DGC8-T39, DGC8-T64, & DGC8-73) and HLA-B57^{(A46E/V97R)}.β2m (DGC8-T54, DGC8-T75 & DGC8-91) on the basis of cell viability (C) and expressed protein titers (D). Table summarizes yield at the different transfection ratios tested.

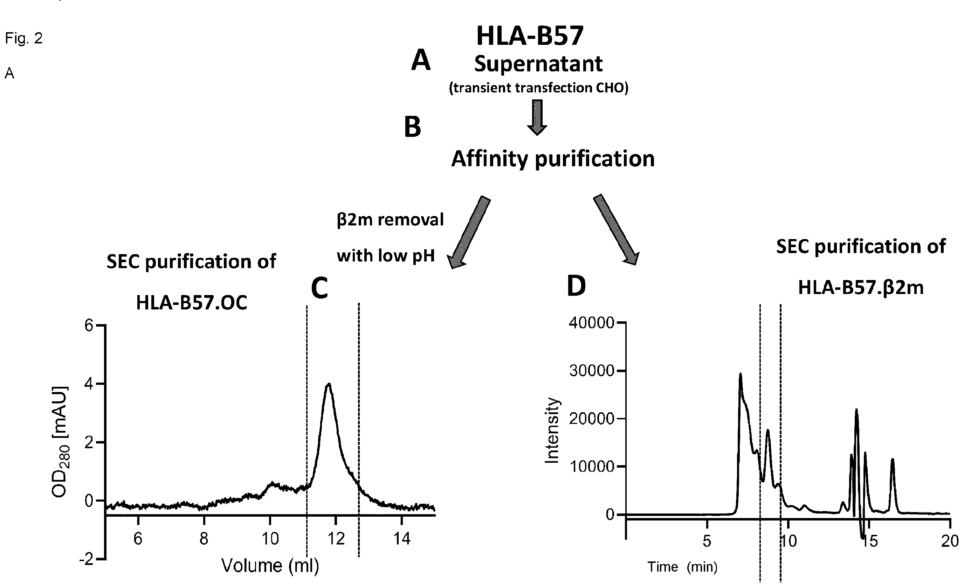

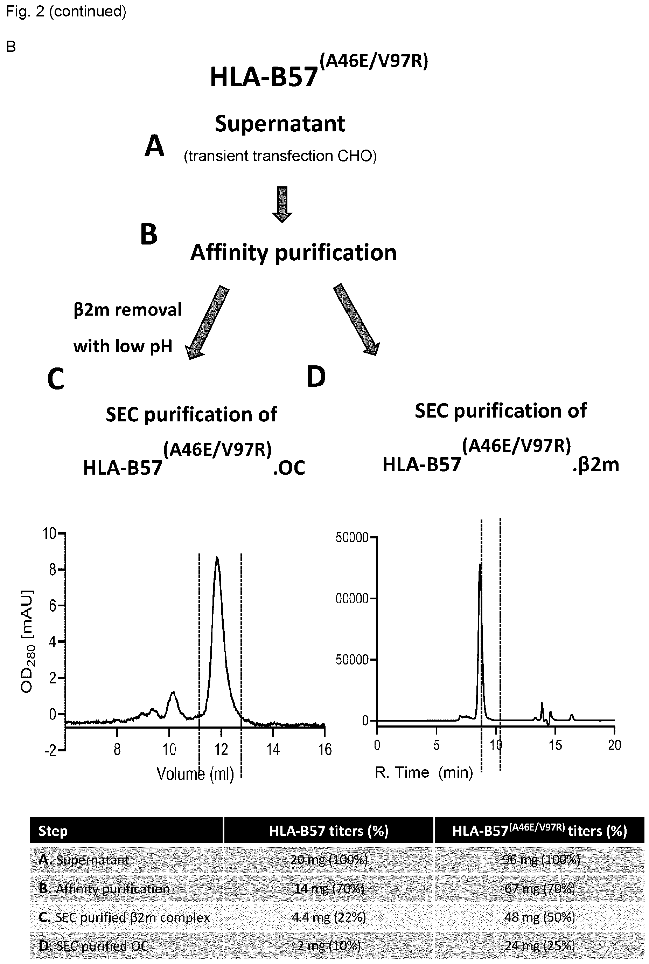

- Fig. 2: shows size exclusion chromatography (SEC) profiles of (A) HLA-B57 and (B) HLA-B57^{(A46E/V97R)} constructs purified in three steps. From CHO supernatant **(A)** affinity purification **(B)** two methods diverge. Open conformer purification is performed with an additional step for B2m removal, followed by SEC (C), and purification of B2m associated HLA-B57 is performed by direct SEC **(D).** Inset summarized table (bottom) demonstrates yield of compounds purified from both constructs. HLA-B57.β2m has high amount of high molecular weight (HMW) species and also low molecular weight species (LMW), with reduced monomer content whereas HLA-B57^{(A46E/V97R)}.β2m has significantly reduced HMW, LWM and high monomer content.

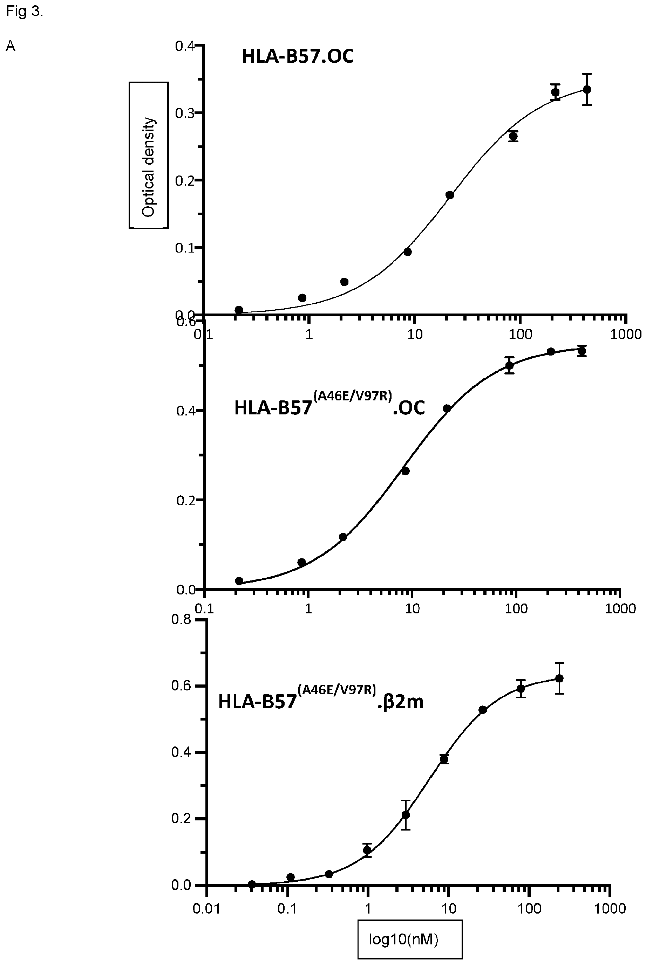

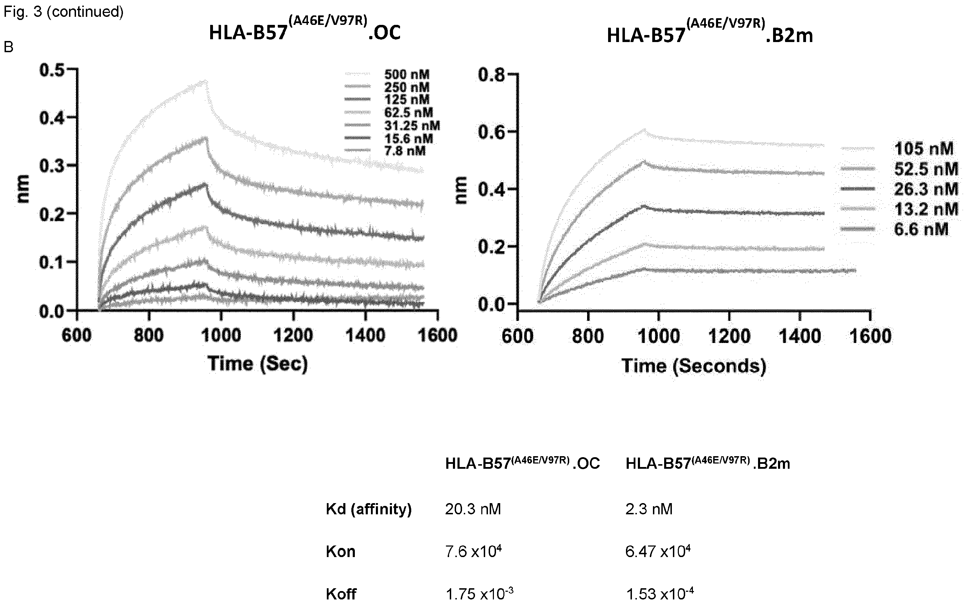

- Fig. 3: (A) shows quantitative estimation of the binding affinities of LILRB2 with HLA-B57.OC, HLA-B57^{(A46E/V97R)}.OC & HLA-B57^{(A46E/V97R)}.β2m measured by ELISA. HLA-B57.OC has an EC50 of 21 nM, HLA-B57^{(A46E/V97R)}.OC EC50 of 8.3 nM, & HLA-B57^{(A46E/V97R)}.β2m EC50 5.72 nM, demonstrating that amino acid substitutions do not reduce the binding of HLA-B57 heavy chain to LILRB2. (B) Quantitative estimation of the binding affinities of LILRB2 with open conformer HLA-B57^{(A46E/V97R)}.OC (Kd=20.3 nM) and HLA-B57(^{A46E/V97R}).β2m (Kd=2.3 nM) measured by Bio-layer interferometry (BLI), confirming improved binding of the B57^{(A46E/V97R)}.β2m associated with β2m compared to open conformer.
- Fig. 4: shows HLA-B57^{(A46E,V97R)}.B2m and open conformer (OC) induced phagocytosis of liquid and solid cancer cells by human primary macrophages. Cancer cell lines derived from the indicated liquid and solid tumors were co-cultured with human primary macrophages, and phagocytosis of tumor cells was measured for 36 hours using IncuCyte live-cell imaging system. Experiments were repeated using at least 2 biologically independent samples. Error bars, SEM of n = 2 biological replicates with each containing 2 technical replicates. Statistical analysis was performed using 2-way ANOVA multiple comparison, followed by Dunnett's post-hoc analysis **p< 0.01, ***p< 0.001, ****p< 0.0001.

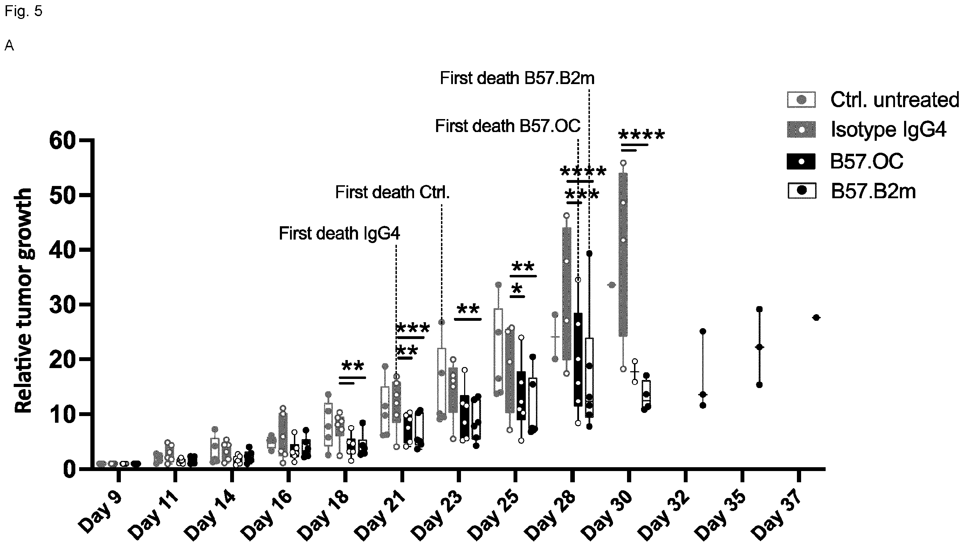

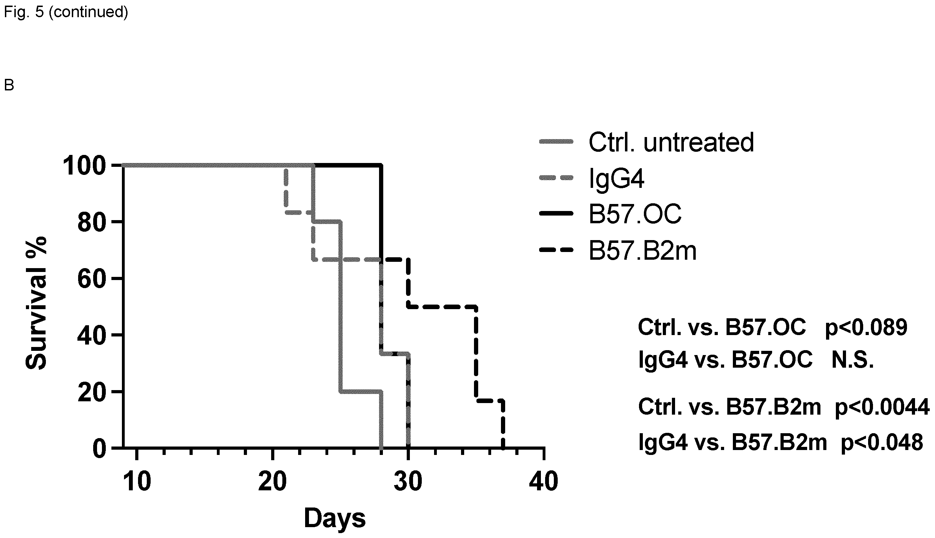

- Fig. 5: shows that open conformer HLA-B57^{(A46E,V97R)}.OC and HLA-B57^{(A46E,V97R}).B2m monotherapy reduce tumor growth, and HLA-B57^{(A46E,V97R}).B2m increase survival in BRGSF-HIS humanized PDX lung cancer mice. A. Relative tumor growth (doubling time of tumor) and (B) survival following HLA-B57^{(A46E,V97R)}.OC or HLA-B57^{(A46E,V97R}).B2m monotherapy. I.p injections were performed every 5 days until end of study; concentration of injected compounds: isotype IgG4 (10 mg/kg), HLA-B57^{(A46E,V97R)}.OC (10 mg/kg), and HLA-B57^{(A46E,V97R)}.B2m (10mg/kg); n = 6. Data in A is plotted as box and whiskers showing all points min. to max. Statistical analysis for A, was performed using multiple comparison 2-way ANOVA (mixed effect model), followed by Tukey's post-hoc analysis where *p< 0.05, **p< 0.01, ***p< 0.001, ****p< 0.0001; Statistical analysis for survival B was performed using a Log-rank (Mantel-Cox) test.

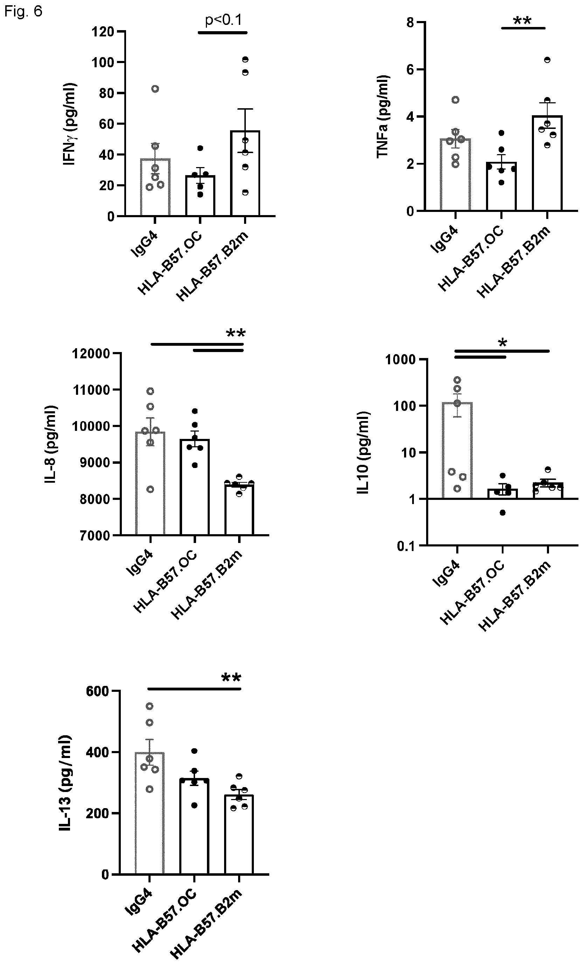

- Fig. 6: shows the analysis of indicated cytokines in the blood of treated BRGSF-HIS humanized PDX lung cancer mice from Fig. 5 measured by V-Plex multiplexed cytokine immunoassay. Statistical analysis was performed using ordinary one-way ANOVA followed by Fischer's post-hoc analysis *p< 0.05, **p<0.01, ***p< 0.001, ****p< 0.0001

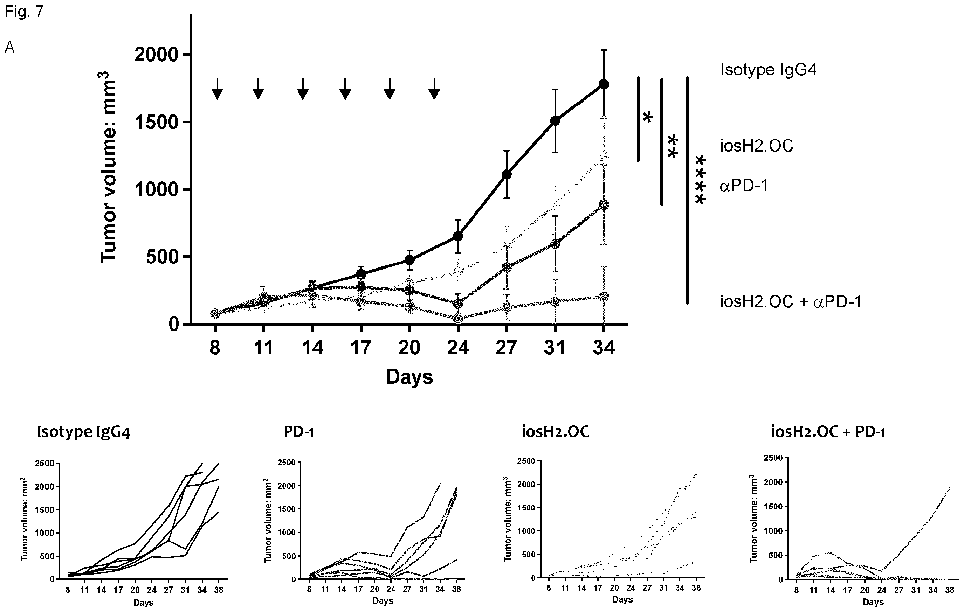

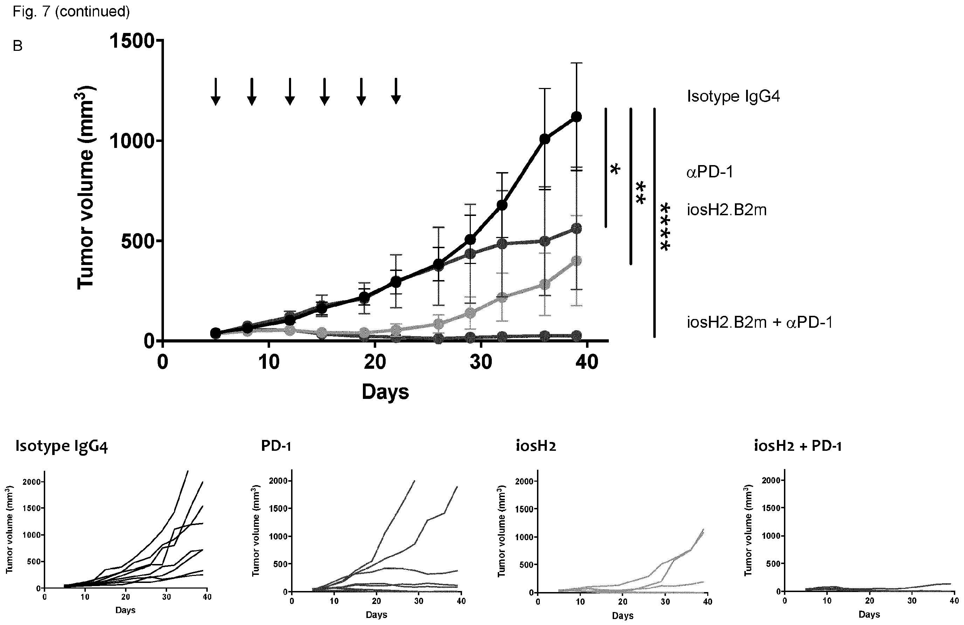

- Fig. 7: shows that monotherapy of and combination therapy with anti-PD-1 checkpoint inhibitors and (A) open conformers (HLA-B57^{(A46E/V97R}.OC) and (B) β2m associated HLA (HLA-B57^{(A46E/V97R)}.β2m) reduces the size of tumors in the C38 murine syngeneic colon carcinoma model. Mean (upper) tumor volume mm³ of indicated treated groups and (lower) spider plot showing individual animals and response to therapy for each group. Tumor volumes are expressed as mean ± SEM and analyzed by two-way ANOVA followed by Bonferroni post-hoc analysis, *p<0.05, **p<0.01, ****p<0.0001.

### Examples

### Example 1. Generation of clone pools

A first HLA fusion protein for medical use in humans was developed by the inventors by linking the heavy chain extracellular domain of the HLA-B57:01:01 polypeptide to an IgG4 Fc polypeptide (SEQ ID NO 002). To increase the yield of this HLA fusion protein, inhibitory amino acids identified in the natural HLA-B57 extracellular domain amino acid sequence were altered by substitution of an alanine (A) residue at position 46 to glutamine (E), and a valine (V) at position 97 to an arginine (R), providing a variant HLA-B67 polypeptide (SEQ ID NO 001). This was fused to the IgG4 polypeptide via a linking peptide of SEQ ID NO 003, to provide a variant HLA-B57 fusion protein (SEQ ID NO 005). cDNA encoding the recombinant HLA-B57^{(A46E/V97R)} fusion protein and the natural HLA-B57-derived fusion protein control, lacking the two mutations, were cloned into commercial expression vectors (Probiogen) downstream of a nucleic acid sequence encoding a secretion signal (SEQ ID NO 004). The vector constructs expressing HLA-B57-Fc & HLA-B57^{(A46E/V97R)}-Fc were co-transfected into Chinese hamster ovary (CHO) cells along with a plasmid comprising a nucleic acid encoding the β2m protein (SEQ NO 006) by microporation (MP) using the NEON Transfection Kit (Life Technologies #MPK10096). CHO-DG44 starter cells were transfected at different ratios of HLA fusion protein to β2m plasmid (4:1, 2:1, 1:1, 1:2). Selected clone pools were grown in standardized shaker flasks and with a defined cell seeding density of 4E5 vc/mL in 125 mL of PBG-CD-C4 supplemented medium including puromycin and methotrexate. Following adjusted selection pression with antibiotics, individual clone pools were selected for analysis. Measurement of viabilities and viable cell densities were performed using the Vi-CELL XR System, and Trypan blue cell exclusion method. Titer quantifications were measured at different time points (days) using an Octet RED machine (ForteBio, a Pall Division) with Protein A biosensors.

### Example 2. Purification of HLA-B57^{(A46E/}^{V97R)}.β2m and β2m removal procedure

Equimolar RNA levels of the natural HLA-B57or altered HLA-B57^{(A46E/V97R)} relative to β2m were confirmed in selected clones cell clones (Fig. 1A). Analysis of clones expressing the HLA-B57 or variant fusion protein demonstrated that HLA-B57.β2m cell viability and titers are significantly lower than HLA-B57^{(A46E/V97R)}.β2m (Fig 1B and C). HPLC analysis of protein collected from supernatants of each demonstrate that HLA-B57.β2m has high amount of high molecular weight (HMW) species and also low molecular weight species (LMW), with reduced monomer content, whereas HLA-B57^{(A46E/V97R)}.β2m has significantly reduced HMW, LWM and high monomer content (Fig 2A and B), demonstrating the increased stability of the variant HLA heavy chain.

The HLA-B57^{(A46E/V97R)}.β2m complex was then isolated from filtered CHO cell supernatants by affinity column purification. Purification of proteins and removal of β2m under acidic conditions was performed as a two-step purification protocol. As a first step, Protein G Sepharose [(4 Fast Flow) Sigma, #GE-17-0618-01)] beads were used to capture HLA-B57^{(A46E/V97R)} associated with β2m from supernatants. After an overnight incubation at 4 degrees on a rocker, the recovered beads were washed in PBS, and subsequently HLA-B57^{(A46E/V97R)} fusion proteins were eluted using standard IgG-Elution Buffer (pH 2.8) (Pierce^{™} IgG Elution Buffer, Thermo Fischer #21004). A second step of size exclusion chromatography-based purification was performed to separate HLA-B57^{(A46E/V97R)} from β2m under acidic conditions, to provide an open conformer HLA fusion protein. A Superdex 10/300 gel filtration column, pre-equilibrated in Sodium Citrate (100 mM, pH 3.0) was used for the separation. An injection of 0.5 ml of the protein at 2.0 mg/ml concentration was applied, and the desired HLA-B57^{(A46E/V97R)}.OC protein peak eluted at 12.7 ml and the peak for β2m eluted at 22.0 ml. Fig. 2 shows the yields of various stages of the process described above, indicating that the separation of β2m to yield an open conformer format HLA fusion protein results in a loss of approximately 50% of the immunomodulatory HLA-B57^{(A46E/V97R)} fusion protein.

### Example 3. Quantification of the interaction of LILRB2 with HLA-B57^{(A46E/}^{V97R)}.OC, and HLA-B57^{(A46E/}^{V97R)}.β2m.

Considering the large loss of yield that accompanies the purification of an open conformer HLA relative to its parent HLA still associated with β2m polypeptide, the inventors went on to dissect the immunological properties most relevant to tumor immunity associated with each HLA-B57^{(A46E/V97R)} format. As a first step, the impact the removal of β2m on from an HLA-B57 IgG4 fusion protein on binding to the innate immune receptor LILRB2 was examined.

The quantification of the affinity of interaction of LILRB2 with HLA-B57.OC, HLA-B57^{(A46E/V97R)}.OC, and HLA-B57^{(A46E/V97R)}.β2m was measured using the enzyme-linked immunosorbent assay (ELISA) method. Flat bottom Pierce^{™} Streptavidin coated high binding capacity 96 well plates (Pierce #15500) were coated with 50 µl of c-terminally biotinylated antigen molecules (LILRB2, BPS Bioscience #100335) immobilized at a final concentration of 5 µg/ml in PBS buffer. PBS and IgG isotype were used as negative controls. A serial dilution of HLA-B57.OC, HLA-B57(^{A46E/V97R}).OC, and HLA-B57^{(A46E/V97R)}.β2m (eight concentration points: 10, 2.5, 1, 0.25, 0.1, 0.025, 0.01, 0.0025 µg/ml) was applied (50 µl) in duplicates. An APC conjugated goat anti-human IgG antibody (Jackson Immuno Research #109-135-098) with 1:100 dilution in TBS (50 µl) was used for detection. Finally, 50 µl TBS in each well was added and a fluorescence scan was performed with APC excitation and emission wavelengths of 650 nm & 660 nm, respectively. Graphpad Prism v9.1.2 and the three-parameter based log (agonist) vs. response model was used to determine the EC50 of the interaction with LILRB2.

The binding of HLA-B57^{(A46E/V97R)}.OC, and HLA-B57^{(A46E/V97R)}.β2 was also assessed by Bio-layer interferometry (BLI). Octet Red 96e (Sartorius) based Bio-Layer Interferometry Technology (BLI) was used for the quantification of binding affinities of HLA fusion proteins with LILRB2. The biotinylated LILRB2 (BPS Bioscience) protein was immobilized on streptavidin (SAXS) biosensors. The biosensors were incubated with increasing concentrations (500, 250, 125, 62.5, 31.25, 15.6 and 7.8nM) of the HLA-B57.OC and (105, 52.5, 26.3, 13.2, and 6.6nM) of HLA-B57^{(A46E/V97R)}:β2m and interaction and reference sensograms were recorded. The data analysis, double reference subtraction and quantification of the binding affinities and kinetic parameters were determined using Data Analysis HT 12.0.2.59 software package and data were fitted locally using a bivalent analyte model (2:1 model) for ligand-analyte reaction.

Together, the binding assays demonstrated improved binding of the variant HLA fusion proteins to LILRB2, as open conformer HLA lacking association with β2m, and particularly when associated with β2m, suggesting the variant HLA heavy chain has high immunomodulatory potential.

### Example 4. Increased killing of tumor cells in vitro

Next, the capacity of the β2m polypeptide to influence HLA-B57^{(A46E,V97R)} IgG4 fusion protein induction of phagocytosis of tumor cells by human primary macrophages was assessed towards liquid (lymphoma, leukemia and myeloma) and solid (breast and lung cancer) cancer cells. Cancer cell lines derived from the indicated liquid and solid tumors were co-cultured with human primary macrophages, and phagocytosis of tumor cells was monitored according to the manufacturer's instructions for 36 hours using IncuCyte live-cell imaging system. Primary human donor-derived monocytes were isolated from PBMCs from healthy donors and differentiated into macrophages by 5-7 days of culture in specific macrophage culture medium. On day 1 post plating compounds were added to wells at 10ug/ml (isotype controls) or 20ug/ml (HLA fusion protein). On day 5-7 post plating, compounds were added once again to the macrophages and two downstream experiments were performed to determine phagocytosis potential of macrophages using live-cell imaging.

Cancer cells were stained with CellTrace^{™} CFSE (ThermoFisher) according to manufacturer's instructions and subsequently 1000 cells/well were plated in flat-bottom 96 well plates (Greiner) together with 1000-5000 primary T cells. Media contained 250nM of Cytotox Red (Sartorius). Live cell imaging was performed using the Incucyte S3 Live-Cell Analysis System (Sartorius). 4 non-adjacent images per well were analysed with Incucyte software v2020C. The CFSE signal was segmented in green objects, and every object was counted as cancer cell. Dead cells were identified with Cytotox signal segmented in red objects. Cancer cell death was detected by colocalization of green and red objects. Results demonstrate that HLA-B57^{(A46E, V97R}).B2m increases the activity of macrophage phagocytosis against cancer cells, while HLA-B57^{(A46E, V97R)}.OC shows slightly reduced activity (Fig 4).

### Example 5 immunomodulation of tumor growth in vivo

HLA-B57^{(A46E,V97R)}.OC and HLA-B57^{(A46E,V97R}).B2m were then each assessed for impact on tumor growth *in vivo* in BRGSF-HIS mice (Rag2*^{-l-}*, IL-2Rγ*^{-l-}*, Flk2*^{-l-}*, bearing a NOD specific SIRPa mutation inhibiting murine endogenous macrophages). These recipients were humanized with intrahepatic injection of purified human umbilical cord blood derived CD34+ cells, and implanted with patient-derived xenograft (PDX) lung cancer cells. These mice comprise all the major human immune cell subsets, including T and B cells, NK cells, and myeloid cells. The non-small cell lung cancer tumors used for xenograft were assessed for expression of the HLA fusion protein target LILRB2 after transplantation in the lung using a commercially available immunohistochemistry antibody, and positive expression following engraftment was confirmed, demonstrating this is an effective model for assessing human LILRB2-mediated immunomodulatory effects.

LU6425 Lung PDX NSCLC tumor fragments were obtained frozen from CrownBio's HuPrime^{®} PDX collection. LU6425 tumor is derived from a western female, age 69. Pathology diagnosis: adenocarcinoma of the lung. Eighteen BRGSF-HIS humanized mice were obtained for efficacy studies, humanization was performed with 6 different HSCs CD34+ donors, with cut-off engraftment of >30% human CD45+ cells (Genoway). LU6425 PDX tumors were expanded in BRGSF mice, PDX fragments are stored frozen in RPMI1640:FBS:DMSO (6:3:1) in liquid nitrogen until use. The fragments were thawed at 37°C for 5 min, rinsed twice in culture media RPM 11640 medium (ref. L0500-500, Dutscher or equivalent). Ten (10) female BRGSF mice were subcutaneously implanted into the left flank with LU6425 tumor fragments.

When tumor volumes of mice from the in vivo amplification phase reached 500-1000 mm3 were surgically excised and tumor fragments (2-3mm3) were subcutaneously implanted into the left flank of eighteen (18) female BRGSF-HIS mice. The day of tumor implantation is considered as day 0 (D0). Once tumors were established, mice received 4 intraperitoneal injections of Flt3 ligand (10 µg per injection) over 7 days. In this protocol, the Flt3 ligand expands the myeloid cell population. Flt3 injections were started 1 day before treatment onset, when tumors reached a mean volume of ∼30- 250mm3. 6 mice each were allocated to IgG4 isotype control, HLA-B57^{(A46E,V97R)}.OC or HLA-B57^{(A46E,V97R}).B2m treatment groups with uniform mean tumor volume between groups. The treatment was administered by injection into the peritoneal cavity (IP). The administration volume was 10 mL/kg adjusted to the most recent individual body weight. Mice were euthanized at a cutoff tumour volume. Animal welfare for this study complies with the UK Animals Scientific Procedures Act 1986 (ASPA) in line with Directive 2010/63/EU of the European Parliament and the Council of 22 September 2010 on the protection of animals used for scientific purposes. All experimental data management and reporting procedures were in strict accordance with applicable Crown Bioscience UK Guidelines and Standard Operating Procedures.

Both HLA fusion protein compounds were injected intraperitoneally every five days for the duration of the experiment. The relative tumor growth (doubling time) was significantly reduced by both HLA-B57^{(A46E, V97R)}.OC and HLA-B57^{(A46E,V97R}).B2m monotherapy (Fig. 5A). However, HLA-B57^{(A46E, V97R}).B2m monotherapy also significantly extended the survival of treated mice vs. controls in this highly relevant cancer model for human immunotherapy (Fig. 5B). The concentration of cytokines in the plasma of treated BRGSF-HIS humanized PDX lung cancer mice collected at terminal stage, and was then assessed using a V-PLEX Proinflammatory Panel 1 Human MSD (MSD). HLA-B57^{(A41E, V97R}).B2m significantly modulated several blood cytokines following therapy in mice, while HLA-B57^{(A46E, V97R)}.OC monotherapy altered only IL-10 levels compared to the control IgG4 treated group, confirming that an HLA fusion protein associated with B2m was a stronger immunomodulator in this cancer model (Fig. 6).

### Example 6 Combination with checkpoint inhibitors

Both B2m-associated and open conformer HLA fusion protein compounds were next tested for their ability to complement checkpoint inhibitor therapy with a PD-1 neutralizing antibody. C38 tumor fragments were injected subcutaneously into the right flanks of syngeneic female C57BL/6 mice. Once the tumor reached ±50 mm³, animals were equally distributed according among groups with equivalent mean tumor volume size. Tumor diameters were measured using a caliper over the course of the study, and volume was calculated according to the formula, *D*/2×*d*² where D and d correspond to the longest and shortest diameter of the tumor in mm, respectively. The experimental design of injection time points of cells and injection of substances was as follows: isotype IgG4 (10mg/Kg) bi-weekly x 3; HLA-B57^{(A46E/V97R)}.OC (5 mg/Kg) bi-weekly x 3; HLA-B57^{(A46E/V97R)}.β2m (5 mg/kg) bi-weekly x 2. Some groups received simultaneous combination treatment with 10mg/kg injections of anti-PD-1 (RMP1-14) In this murine model, both compounds provided some advantage to counter tumor growth, however pharmaceutical compositions comprising B2m-associated HLA fusion protein more effectively controlled tumor growth in combination with PD-1 in all animals in the group, whereas a single instance of tumor escape and growth was observed in the equivalent open conformer group (Fig. 7).

### Summary

Unexpectedly, the data demonstrated that an HLA-B57^{(A46/V97)} IgG4 fusion protein was equally effective at impeding tumor growth in vivo either with, or without the presence of β2m. To the inventor's knowledge, this is the first time that the utility of β2m association with human HLA fusion proteins has been examined in comprehensive models examining the human lymphoid and myeloid cells which are an important target for HLA fusion protein function both *in vitro* and *in vivo.* As HLA open conformer fusion proteins have considerably higher yields due to the shorter production process (Fig. 2), these finding suggests it may be desirable to use pharmaceutical composition comprising an open conformer HLA molecule and a β2m molecule according to the invention in antineoplastic therapeutic settings including, but not limited to epithelial cancers such as lung cancer, colon cancer, and breast cancer, and blood cell malignancies such as myeloma, lymphoma, and leukemia.

### Sequences:

SEQ ID NO 001 variant HLA-B57:01 extracellular domain A46E V97R (artificial) 
GSHSMRYFYTAMSRPGRGEPRFIAVGYVDDTQFVRFDSDAASPRMEPRAPWIEQEGPEYWDGETRNMK
ASAQTYRENLRIALRYYNQSEAGSHIIQRMYGCDVGPDGRLLRGHDQSAYDGKDYIALNEDLSSWTAA
DTAAQITQRKWEAARVAEQLRAYLEGLCVEWLRRYLENGKETLQRADPPKTHVTHHPISDHEATLRCW
ALGFYPAEITLTWQRDGEDQTQDTELVETRPAGDRTFQKWAAVVVPSGEEQRYTCHVQHEGLPKPLTL
RWEPSSQS
SEQ ID NO 002 Optimized IgG4 Fc (artificial) 
ESKYGPPCPPCPAPEFLGGPSVFLFPPKPKDTLMISRTPEVTCVVVDVSQEDPEVQFNWYVDGVEVHN
AKTKPREEQFNSTYRVVSVLTVLHQDWLNGKEYKCKVSNKGLPSSIEKTISKAKGQPREPQVYTLPPS
QEEMTKNQVSLTCLVKGFYPSDIAVEWESNGQPENNYKTTPPVLDSDGSFFLYSRLTVDKSRWQEGNV
FSCSVMHEALHNHYTQKSLSLSLG
SEQ ID NO 003 peptide linker (artificial) GGGGSGGGGS
SEQ ID NO 004 secretory signal (artificial) AAAMNFGLRLIFLVLTLKGVQC
SEQ ID NO 005 variant HLA-B57:01 extracellular domain A46E V97R IgG4 fusion protein (artificial) 
GSHSMRYFYTAMSRPGRGEPRFIAVGYVDDTQFVRFDSDAASPRMEPRAPWIEQEGPEYWDGETRNMK
ASAQTYRENLRIALRYYNQSEAGSHIIQRMYGCDVGPDGRLLRGHDQSAYDGKDYIALNEDLSSWTAA
DTAAQITQRKWEAARVAEQLRAYLEGLCVEWLRRYLENGKETLQRADPPKTHVTHHPISDHEATLRCW
ALGFYPAEITLTWQRDGEDQTQDTELVETRPAGDRTFQKWAAVVVPSGEEQRYTCHVQHEGLPKPLTL
RWEPSSQSGGGGSGGGGSESKYGPPCPPCPAPEFLGGPSVFLFPPKPKDTLMISRTPEVTCVVVDVSQ
EDPEVQFNWYVDGVEVHNAKTKPREEQFNSTYRVVSVLTVLHQDWLNGKEYKCKVSNKGLPSSIEKTI
SKAKGQPREPQVYTLPPSQEEMTKNQVSLTCLVKGFYPSDIAVEWESNGQPENNYKTTPPVLDSDGSF
FLYSRLTVDKSRWQEGNVFSCSVMHEALHNHYTQKSLSLSLG
SEQ ID NO 006. B2m (homo sapiens)

## Claims

1. A pharmaceutical composition for use in the treatment of a malignant neoplastic disease, said composition comprising:
a. an HLA fusion protein comprising:
i. a human leukocyte antigen (HLA) heavy chain polypeptide selected from:
• an extracellular domain of an HLA heavy chain, particularly an HLA heavy chain selected from HLA-B57, HLA-C08, HLA-A25, HLA-B58, HLA-B27, HLA-A30, HLA-B53, or HLA-C12; or
• a variant of said extracellular domain of an HLA heavy chain, wherein said variant is **characterized by** a sequence similarity of at least (≥) 95%, particularly ≥98%, and a similar biological activity;
ii. an immunoglobulin crystallizable fragment (Ig Fc) polypeptide, particularly an IgG Fc polypeptide, more particularly an IgG4 Fc polypeptide; and
b. a beta 2 microglobulin (B2m) polypeptide.

2. The pharmaceutical composition for use according to claim 1, wherein the HLA fusion protein is non-covalently associated with the B2m polypeptide.

3. The pharmaceutical composition for use according to claim 1 or 2, wherein the HLA fusion protein is non-covalently associated with the B2m polypeptide at a ratio of between 3:5 to 7:5, particularly between 4:5 to 6:5, more particularly at a ratio of about 1.

4. The pharmaceutical composition for use according to any one of the claims 1 to 3, wherein the HLA heavy chain polypeptide is a variant of the extracellular domain of HLA-B57,
and wherein HLA heavy chain polypeptide is **characterized by** an E at position 46, and an R at position 97.

5. The pharmaceutical composition for use according to any one of the claims 1 to 4, wherein the HLA heavy chain polypeptide comprises, or essentially consists of, the sequence SEQ ID NO 001.

6. The pharmaceutical composition for use according to any one of the claims 1 or 5, wherein the HLA fusion protein comprises:
a. the HLA heavy chain polypeptide as specified in any one of the claims 1 to 5;
b. an IgG Fc polypeptide, particularly an IgG4 F polypeptide, more particularly an IgG4 Fc polypeptide with the sequence SEQ ID NO 002; and/or
c. a peptide linker connecting the HLA heavy chain polypeptide to the IgG Fc polypeptide, particularly a peptide linker between 5 and 20 amino acids in length, more particularly a peptide linker with the sequence SEQ ID NO 003;
and wherein optionally, the HLA fusion protein further comprises:
d. a secretory signal, particularly wherein the secretory signal is 16 to 30 amino acids in length, more particularly wherein the secretory signal is removed by cleavage during the process of secretion from the cell, still more particularly wherein the secretory signal has the sequence SEQ ID NO 004.

7. The pharmaceutical composition for use according to any one of the claims 1 to 6, wherein the HLA fusion protein comprises, or essentially consists of, the sequence designated SEQ ID NO 005.

8. The pharmaceutical composition for use according to any one of the claims 1 to 7, wherein the HLA fusion protein is in the form of a dimer, said dimer comprising, or essentially consisting of a first HLA monomer and a second HLA monomer;
- wherein the first HLA monomer essentially consists of a first HLA fusion protein as specified in any one of the claims 1 to 7, and a first B2m polypeptide; and
- wherein the second HLA monomer essentially consists of a second HLA fusion protein as specified in any one of the claims 1 to 7, and a second B2m polypeptide;
particularly wherein the first and the second HLA monomer are identical.

9. The pharmaceutical composition for use according to any one of the claims 1 to 8, wherein the pharmaceutical composition is administered prior to, in combination with, or subsequent to a checkpoint inhibitory agent.

10. A checkpoint inhibitory agent for use in the treatment of a malignant neoplastic disease, wherein the checkpoint inhibitory agent is administered prior to, in combination with, or subsequent to the pharmaceutical composition for use according to any one of the claims 1 to 9.

11. The pharmaceutical composition for use according to any one of the claims 1 to 9, or the checkpoint inhibitory agent for use according to claim 10, wherein said checkpoint inhibitory agent is capable of binding to one of CTLA-4, PD-1, PD-L1, or PD-L2 with a dissociation constant of at least 10⁻⁷ M⁻¹, particularly wherein the checkpoint inhibitory agent is selected from an antibody, an antibody fragment, or an antibody-like molecule.

12. The pharmaceutical composition for use according to any one of the claims 1 to 9, or 11, or the checkpoint inhibitory agent for use according to claim 10 or 11, wherein said checkpoint inhibitory agent is provided in a dosage form suitable for systemic delivery.

13. A pharmaceutical composition for use according to any one of the claims 1 to 9, 11 or 12, wherein the malignant neoplastic disease is
a. a blood-cell derived cancer, particularly a blood cell derived cancer selected from lymphoma, leukemia, or myeloma, or
b. a solid tumor, particularly a lung, breast, or colon cell-derived solid tumor.

14. The checkpoint inhibitory agent for use according to any one of the claims 10 to 12, wherein the malignant neoplastic disease is selected from colon cancer, breast cancer, pancreatic cancer, or melanoma.
